# EUROPEAN PATENT APPLICATION

(11) **EP 4 000 668 A1**
(43) Date of publication of application: **25.05.2022**
(21) Application number: 21207347.2
(22) Date of filing: 10.11.2021
(51) Int. Cl.: A61M 5/158, A61M 5/32, A61M 5/142

(54) **INSERTION BASED ON HELICAL TRACKS**

(30) Priority: 11.11.2020 US 202063112548 P; 08.10.2021 US 202117496876
(71) Applicant: Medtronic MiniMed, Inc., Northridge, CA 91325-1219 (US)
(72) Inventor: Yavorsky, Matthew William, Granada Hills, 91344 (US)
(74) Representative: Zimmermann & Partner Patentanwälte mbB

(57) **Abstract**

A torsional insertion mechanism includes a torsion spring configured to rotate a bushing between a first spring position and a second spring position and an insertion assembly configured to move from a first insertion position to a second insertion position in response to rotation of the bushing. The insertion assembly includes a cannula and a captive introducer needle configured to pierce tissue.

## Description

### CROSS-REFERENCE TO RELATED APPLICATIONS

The present application claims the benefit of and priority to U.S. Provisional Patent Application Serial No. 63/112,548, filed on November 11, 2020, the entire content of which being hereby incorporated by reference.

### TECHNICAL FIELD

This disclosure relates generally to insertion devices, and more particularly, to insulin infusion systems including torsional insertion mechanisms for cannula insertion.

### BACKGROUND

A person may use insulin therapy to manage type I or type II diabetes. Insulin therapy may include use of insulin infusion systems for delivering or dispensing insulin. An insulin infusion system may include an infusion device which typically includes a small motor and drive train components configured to deliver insulin from a reservoir into the body of a person, e.g., via a percutaneous needle or a cannula placed in the subcutaneous tissue. Insulin infusion systems may facilitate management of diabetes for some persons.

### SUMMARY

This disclosure relates generally to insertion devices, and more particularly, to torsional insertion mechanisms.

In accordance with aspects of the present disclosure, a torsional insertion mechanism includes a torsion spring configured to rotate a bushing between a first spring position and a second spring position, and an insertion assembly configured to move from a first insertion position to a second insertion position in response to the rotation of the bushing. The insertion assembly includes a captive introducer needle configured to pierce tissue, and a cannula.

In an aspect of the present disclosure, the bushing may include an inner surface including an angled ramp.

In another aspect of the present disclosure, the insertion assembly may include a tubular boss configured to contact the angled ramp and move from a first boss position to a second boss position in response to the rotation of the bushing.

In yet another aspect of the present disclosure, the angled ramp may contact the insertion assembly.

In yet a further aspect of the present disclosure, the angled ramp may include a track that pushes the captive introducer needle down and/or pulls the captive introducer needle out in response to the rotation of the bushing.

In an aspect of the present disclosure, the torsional insertion mechanism may further include a stop member configured to selectively prevent rotation of the bushing.

In another aspect of the present disclosure, the stop member may be configured to move from a first stop position to prevent rotation of the bushing by engaging a stop recess in an outer surface of the bushing, to a second stop position to enable rotation of the bushing by disengaging the stop recess of the bushing.

In yet another aspect of the present disclosure, the insertion assembly may further include a cannula carrier configured to capture the cannula, a needle guide configured to guide the cannula in the cannula carrier, and a fluid flow path that passes through the needle guide to the cannula in the cannula carrier. The fluid flow path is configured for fluid communication between the cannula and a medical reservoir.

In a further aspect of the present disclosure, the needle guide may further include a tubular boss extended from a bottom of the needle guide. The cannula carrier may include a bore. The cannula may be captured in a radial gap between the bore in the cannula carrier and the tubular boss.

In an aspect of the present disclosure, the introducer needle and the cannula may be configured to move from a first needle position to a second needle position in response to the insertion assembly moving from the first insertion position to the second insertion position.

In another aspect of the present disclosure, the introducer needle may be configured to move to the first needle position from the second needle position, and the cannula remains in the second needle position in response to the insertion assembly moving from the second insertion position to a third insertion position.

In accordance with aspects of the disclosure, an infusion pump system includes a torsional insertion mechanism, a medical reservoir in fluid communication with an insertion assembly of the torsional insertion mechanism, and a motor. The torsional insertion mechanism includes a torsion spring configured to rotate a bushing between a first spring position and a second spring position, an insertion assembly configured to move from a first insertion position to a second insertion position in response to the rotation of the bushing, and a stop member configured to enable and/or disable rotation of the bushing. The motor is configured to engage and/or disengage the stop member.

In yet another aspect of the present disclosure, the bushing may include an inner surface including an angled ramp.

In a further aspect of the present disclosure, the insertion assembly may include a tubular boss configured to contact the angled ramp and move from a first boss position to a second boss position in response to the rotation of the bushing.

In yet a further aspect of the present disclosure, the angled ramp may contact the insertion assembly.

In an aspect of the present disclosure, the insertion assembly may include a captive introducer needle, and a cannula configured for insertion in tissue in response to rotational motion of the bushing.

In another aspect of the present disclosure, the angled ramp may include a track that pushes the captive introducer needle down and/or pulls the captive introducer needle out in response to the rotation of the bushing.

In an aspect of the present disclosure, a method for operating a torsional inserter of an insulin infusion system is disclosed. The method includes rotating a bushing between a first spring position and a second spring position by a torsion spring, moving an insertion assembly from a first insertion position to a second insertion position in response to the rotation of the bushing, and moving an introducer needle and a cannula from a first needle position to a second needle position in response to moving from a first insertion position to a second insertion position.

In an aspect of the present disclosure, the method may further include moving the introducer needle and the cannula from a first needle position to a second needle position in response to the insertion assembly moving from the first insertion position to the second insertion position.

In another aspect of the present disclosure, the method may further include moving the introducer needle to the first needle position from the second needle position, and the cannula remaining in the second needle position in response to the insertion assembly moving from the second insertion position to a third insertion position.

### BRIEF DESCRIPTION OF THE DRAWINGS

The above and other aspects and features of the disclosure will become more apparent in view of the following detailed description when taken in conjunction with the accompanying drawings wherein like reference numerals identify similar or identical elements.
FIG. 1 is an illustration of an exemplary torsional insertion mechanism, in accordance with aspects of the disclosure;
FIG. 2 is an illustration of the torsional insertion mechanism of FIG. 1 showing an upper housing of the torsional insertion mechanism removed, in accordance with aspects of the disclosure;
FIG. 3 is a cross-sectional view of the torsional insertion mechanism of FIG. 1 illustrating components of the torsional insertion mechanism and showing the insertion assembly of the insertion mechanism in a first position, in accordance with aspects of the disclosure;
FIGS. 4A and 4B are side views of a split bushing of the torsional insertion mechanism of FIG. 1, in accordance with aspects of the disclosure;
FIG. 5 is a top view of the split bushing of FIGS. 4A and 4B, in accordance with aspects of the disclosure;
FIG. 6 is a perspective view of the split bushing of FIGS. 4A and 4B with a needle carrier disposed therein, in accordance with aspects of the disclosure;
FIG. 7 is a perspective view of the split bushing of FIGS. 4A and 4B disposed in the lower housing of the torsional insertion mechanism, in accordance with aspects of the disclosure;
FIG. 8 is a cross-sectional view of the torsional insertion mechanism of FIG. 1 showing a plug installed in the lower housing, in accordance with aspects of the disclosure;
FIG. 9 is a cross-sectional view of the torsional insertion mechanism of FIG. 1 showing the plug of FIG. 8 removed from the lower housing, in accordance with aspects of the disclosure;
FIG. 10A is a side view of the torsional insertion mechanism of FIG. 1 showing a stop member being engaged with the bushing of the torsional insertion mechanism, in accordance with aspects of the disclosure;
FIG. 10B is a side view of the torsional insertion mechanism of FIG. 1 showing the stop member being disengaged with the bushing of the torsional insertion mechanism, in accordance with aspects of the disclosure;
FIG. 11 is a side view of the torsional insertion mechanism of FIG. 1 showing the insertion assembly of the insertion mechanism in a first position, in accordance with aspects of the disclosure;
FIG. 12A and 12B are progressive cutaway side views of the torsional insertion mechanism of FIG. 1 showing the rotation of the bushing, in accordance with aspects of the disclosure;
FIG. 13A and 13B are progressive side views of the torsional insertion mechanism of FIG. 1 showing the rotation of the bushing, in accordance with aspects of the disclosure;
FIG. 14 is a bottom perspective view of the torsional insertion mechanism of FIG. 1 showing a snap mechanism holding a cannula carrier and needle guide in place, in accordance with aspects of the disclosure;
FIG. 15 is a cutaway side view of the torsional insertion mechanism of FIG. 12B showing a fluid delivery path, in accordance with aspects of the disclosure;
FIGS. 16A and 16B are progressive perspective views of the torsional insertion mechanism of FIG. 1, showing the bushing rotating from a second position to a third position; and
FIG. 17 is a perspective side view of an exemplary infusion pump system, in accordance with aspects of the disclosure.

### DETAILED DESCRIPTION

This disclosure relates generally to insertion devices, and more particularly, to torsional insertion mechanisms for insulin infusion systems.

Although the disclosure may be described primarily with respect to cannula insertion for insulin infusion systems, the scope of the disclosure is equally applicable to sensors or other devices which include cannula, needles, or the like, that are at least partially implantable.

As used herein, "exemplary" does not necessarily mean "preferred" and may simply refer to an example unless the context clearly indicates otherwise.

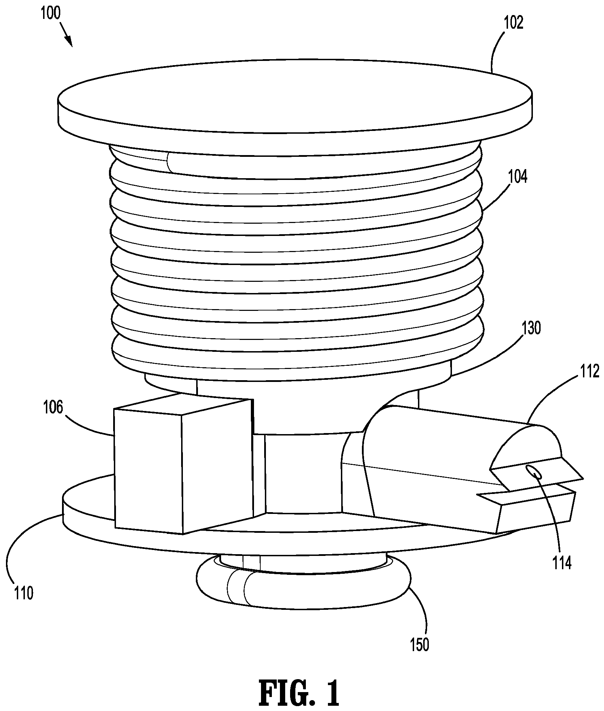

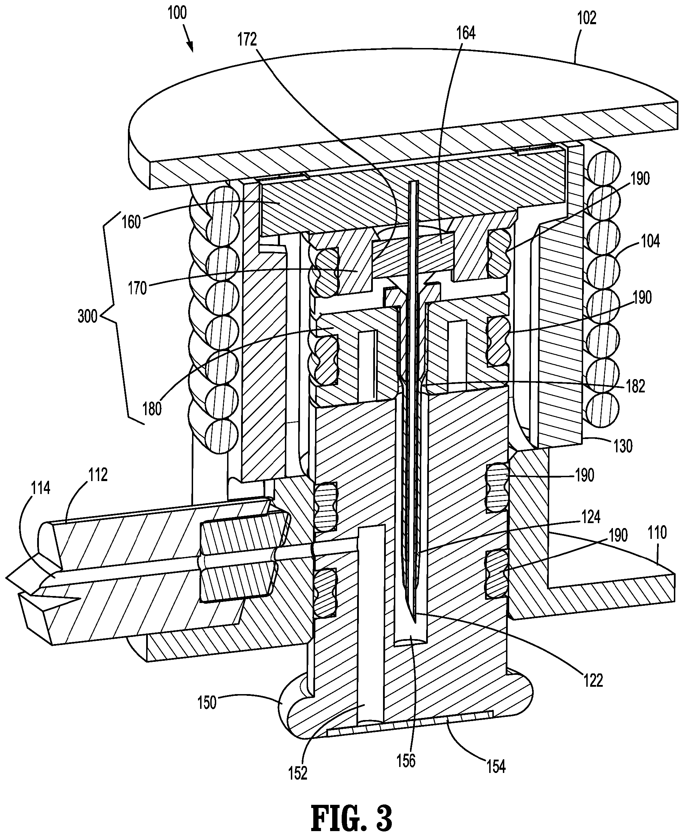

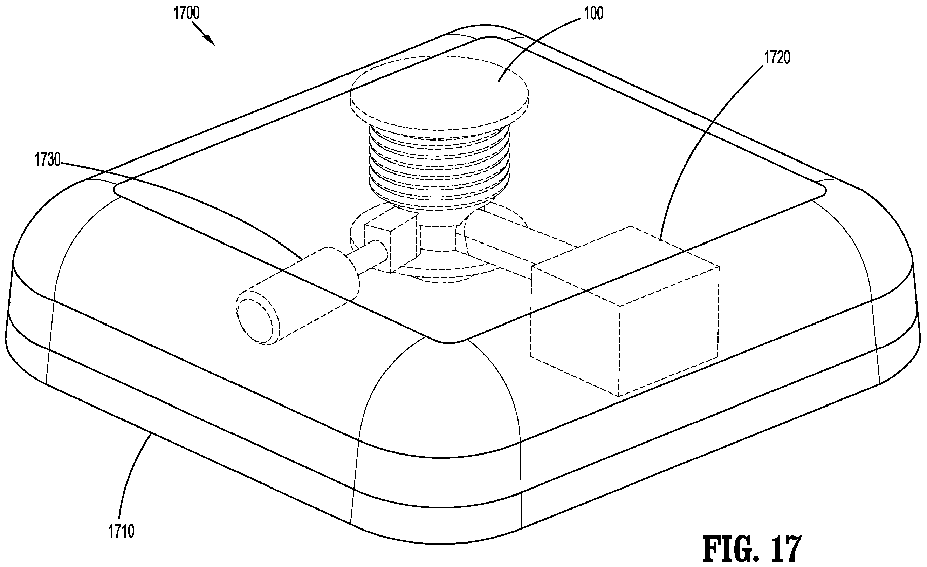

Referring to FIGS. 1-3, an exemplary torsional insertion mechanism 100, of an exemplary infusion pump system 1700 (see FIG. 17) configured for cannula insertion, is shown. The torsional insertion mechanism 100 generally includes an upper housing 102, a torsion spring 104, a bushing 130, a lower housing 110, and an insertion assembly 300 configured to pierce tissue using a captive introducer needle 122 and insert a cannula 124 (FIG. 3) into subcutaneous tissue. In aspects, the torsional insertion mechanism 100 may further include a pull-before-use plug (PGUP) 150 configured to hold the components of the torsional insertion mechanism 100 stable during shipping. The PGUP 150 may also provide a means of occluding the fluid flow path for pump setup. In aspects, the PGUP 150 may incorporate a semi-permeable membrane to allow transmission of ethylene oxide gas for sterilization. The upper housing 102 is fixedly attached to a top surface of the bushing 130.

The torsion spring 104 is slidably disposed around the bushing 130. The torsion spring 104 is configured to rotate the bushing 130 between a first spring position and a second spring position. The torsion spring 104 includes a proximal portion 104a and a distal portion 104b. The proximal portion 104a of the torsion spring 104 may be retained in a recess 136 at the top surface 136b of the bushing 130. The torsion spring 104 may be pre-loaded (e.g., placed under tension) prior to installation in an infusion pump system 1700 (FIG. 17) such that the torsion spring 104 stores potential energy for later use.

In aspects, the torsional insertion mechanism 100 may further include a stop member 106 configured to selectively prevent rotation of the bushing 130. The stop member 106 may be configured to move from a first stop position to prevent rotation of the bushing 130 by engaging the stop recess 133 of the bushing 130 to a second stop position to enable rotation of the bushing 130 by disengaging the stop recess 133 of the bushing 130. The stop member 106 may be used to prevent rotation of the bushing 130 until cannula 124 insertion is desired. It is contemplated that any of a variety of triggering techniques may be used to move the stop member 106 out of the way and allow the torsion spring 104 to impart rotational motion to the bushing 130.

Referring to FIG. 3, a cross-sectional view of the insertion assembly 300 of the torsional insertion mechanism 100 is shown. The insertion assembly 300 is configured to move from a first insertion position to a second insertion position in response to the rotation of the bushing 130. The insertion assembly 300 generally includes a needle carrier 160, a needle guide 170, a cannula carrier 180, and one or more tubular bosses 162a, 162b. The first insertion position of insertion assembly 300 may be a proximal position, and the second insertion position of insertion assembly 300 may be a distal position.

The needle carrier 160 of insertion assembly 300 generally includes a captive introducer needle 122. The introducer needle 122 projects outwardly and axially from the bottom of the insertion assembly 300 and is configured to pierce the skin of a user and to enable the cannula 124 to penetrate or extend through the skin of the user.

The needle guide 170 of insertion assembly 300 generally includes one or more radial seals 190 around a perimeter of the needle guide 170 and a captive elastomer septum 164 that seals around the introducer needle 122. In the figures, the one or more radial seals 190 are depicted as redundant dual lobed seals. However, it should be appreciated that one or more single lobed seals can be used instead. This includes the benefit of enabling the reduction in height and/or size of the insertion assembly 300.

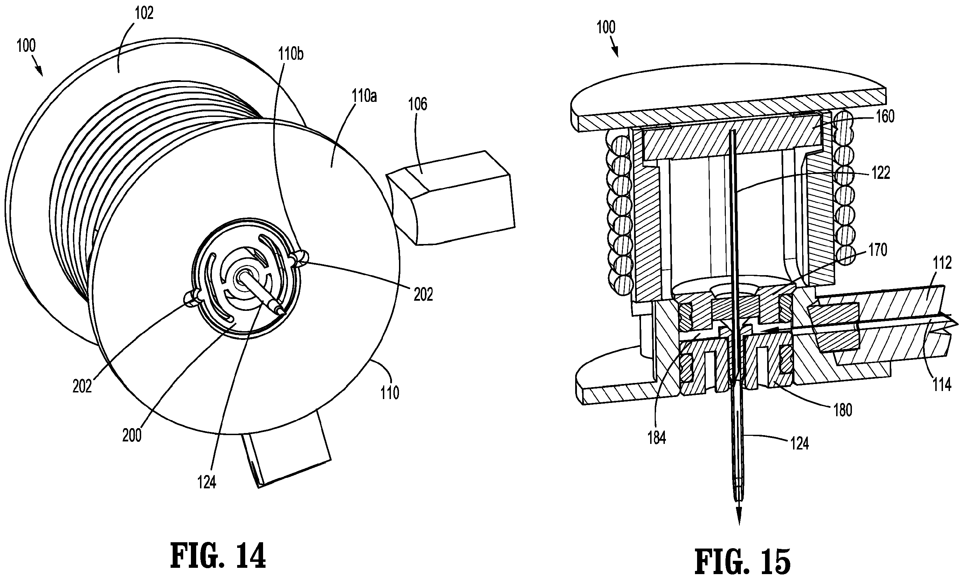

The cannula carrier 180 of insertion assembly 300 generally includes a cannula 124 configured for fluid communication with a medical reservoir 1720 (FIG. 17) configured for holding a fluid medicament (e.g., insulin), one or more radial seals 190 around the perimeter of the cannula carrier 180 configured for sealing the cannula carrier 180, and a through bore 182 configured for permitting the cannula and introducer needle 122 to pass therethrough. In aspects, the cannula carrier 180 may be secured to the needle guide 170 through a press-fit engagement with the cannula 124, via glue, snaps, welding, and/or other suitable method of attachment. A fluid flow path 184 (FIG. 15) may be defined which passes through the needle guide 170 and to the cannula 124 in the cannula carrier 180. The cannula 124 may be captured in a radial gap defined between the cannula carrier 180 and the tubular boss(es) 162a, 162b of the needle guide 170.

The insertion assembly 300 is configured to move up and/or down or translate axially relative to a longitudinal axis defined by the cannula 124 or cannula carrier 180. In operation, during insertion of the cannula 124 into the tissue or through the skin of the user, the needle carrier 160 may push downward (e.g., in a direction toward the skin of the patient) or act on the needle guide 170, which may push down or act on the cannula carrier 180. In operation, the needle carrier 160 may also pull up (e.g., in a direction away from the skin of the user), to a third introducer position, to at least partially retract the introducer needle 122 through the septum 164 of the needle guide 170.

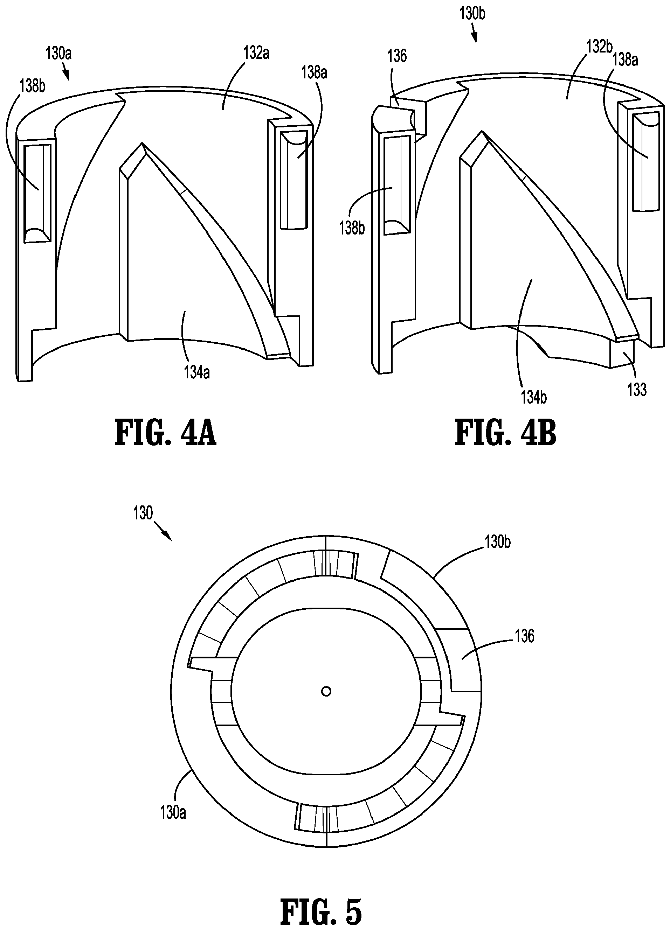

Referring to FIGS. 4A-B, 5, and 6, a bushing 130 of the torsional insertion mechanism 100 is shown. The bushing 130 is disposed around the insertion assembly 300. The bushing 130 is generally tubular in shape and includes an inner surface 132a, 132b with one or more angled ramps 134a, 134b disposed thereon. The angled ramps 134a, 134b may be protrusions or may be one or more recesses configured to receive a boss 162a, 162b of the insertion assembly 300. The angled ramps 134a, 134b include a track 132c that pushes the captive introducer needle 122 down (e.g., toward the skin of the patient) and/or pulls the captive introducer needle 122 out (e.g., away from the skin of the patient) in response to the rotation of the bushing 130. The angled ramps 134a, 134b of the bushing 130 may include any suitable angle of attack (e.g., pitch) to enable rotation of the bushing 130 a suitable number of degrees. For example, with an angle of attack or pitch of about 50 degrees, the rotation of the bushing 130 may be about 180 degrees. However, it should be appreciated that other helical angles or pitches, and other degrees of rotation, are also contemplated. For example, ramp angles or pitches may be used that may cause anywhere from 90 degrees to 360 degrees of rotation.

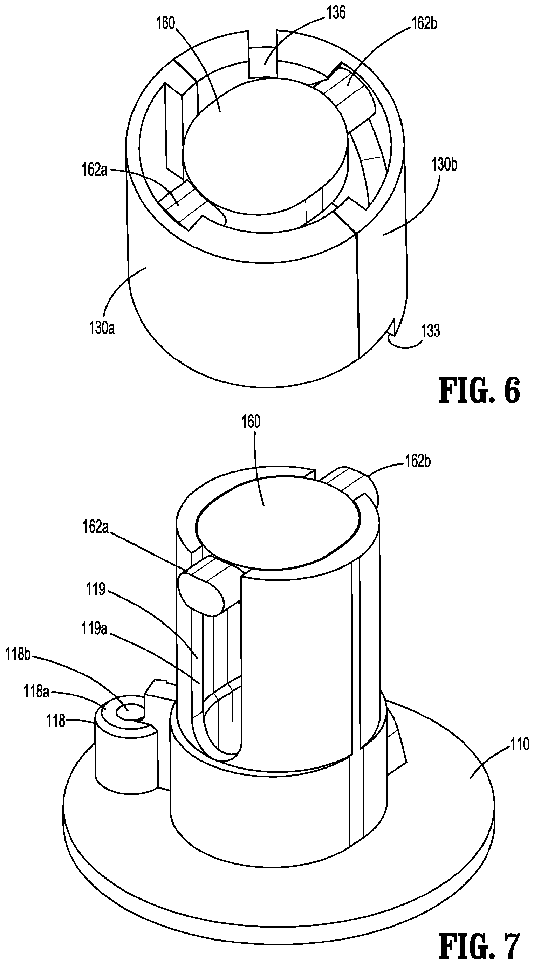

As illustrated in FIG. 7, the tubular bosses 162a, 162b of the needle guide 170 pass through the radially oriented slots 119a of the lower housing 110. In aspects, the tubular bosses 162a, 162b may be integrated into the needle carrier 160. In aspects, the tubular bosses 162a, 162b may be bonded to the introducer needle 122.

In aspects, the bushing 130 may be a split bushing. The bushing 130 includes an outer surface that may include a stop recess 133 that is configured to receive a stop member 106 (FIG. 1) for enabling or disabling rotation of the bushing 130. The angled ramps 134a, 134b of the bushing 130 may contact the insertion assembly 300, which is configured to move up and/or down between a first insertion position and a second insertion position as the bushing 130 rotates. In aspects, the first insertion position is a proximal insertion position (e.g., position further away from the skin of the patient) and the second insertion position is a distal position (e.g., position closer to the skin of the patient).

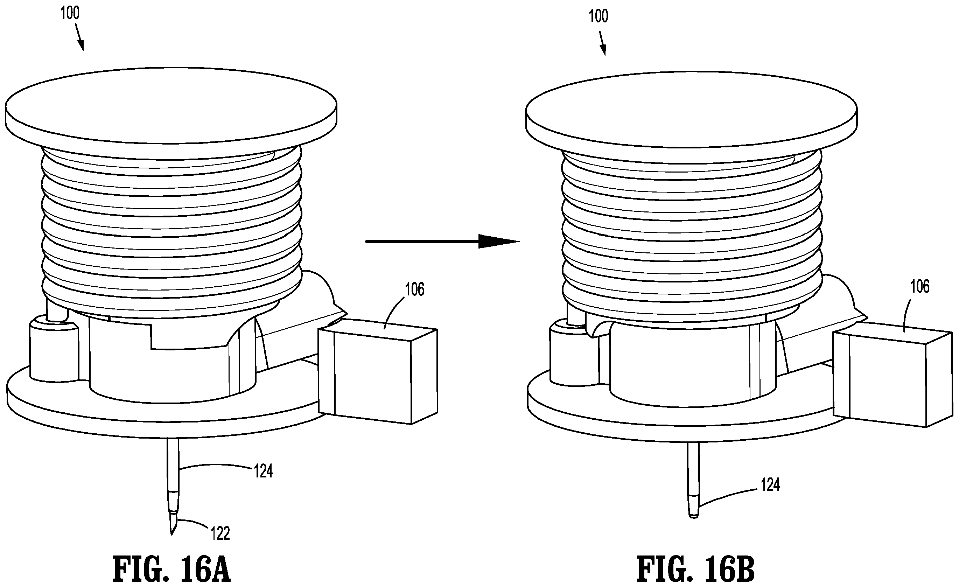

With continued reference to FIG. 7, the lower housing 110, of the torsional insertion mechanism 100, is shown. The lower housing 110 includes a boss 118 configured to retain the distal portion 104b of the torsion spring 104. The boss 118 may include a rotational stop 118' extending from the boss 118. The boss 118 includes a bore 118b in a top surface 118a thereof for retaining the distal portion 104b of the torsion spring 104. The rotational stop 118' is configured for stopping the rotation of the bushing 130 at a third boss position (FIG. 16B) in reaction to the stop recess 133 of the bushing 130 contacting the rotational stop 118'.

The lower housing 110 further includes side walls 119. The side walls 119 include slots 119a defined therein, which are configured to constrain the insertion assembly 300 to vertical motion (e.g., in the direction of insertion and/or retraction) in response to the rotational motion of the bushing 130. The slots 119a are configured for receipt of tubular bosses 162a, 162b. It is contemplated that the slots extend through the side walls 119 and/or may be recesses in the side walls 119.

The lower housing 110 also includes a fluid supply member 112 configured for fluid communication with a medical reservoir 1720 (FIG. 17). The fluid supply member 112 defines a flow path 114 (e.g., a through bore) configured for fluid communication with a medical reservoir 1720 (FIG. 17) and the cannula 124.

As illustrated in FIGS. 6 and 7, the tubular bosses 162a, 162b are configured to contact the angled ramps 134a, 134b of the bushing 130 and move from a first boss position to a second boss position in response to a rotation of the bushing 130. For example, as the bushing 130 rotates, in response to the rotational motion of the torsion spring 104, the angled ramps 134a, 134b of the bushing 130 may contact the tubular bosses 162a, 162b of the needle carrier 160. As the needle carrier 160 is constrained to vertical or axial motion, the rotation of the bushing 130 may push the introducer needle 122 and the cannula 124 in a downward direction and into the tissue of the patient. Continued rotation of the bushing 130, in the same direction, to a third bushing position may pull the introducer needle 122 of the tissue out while the cannula 124 remains in place.

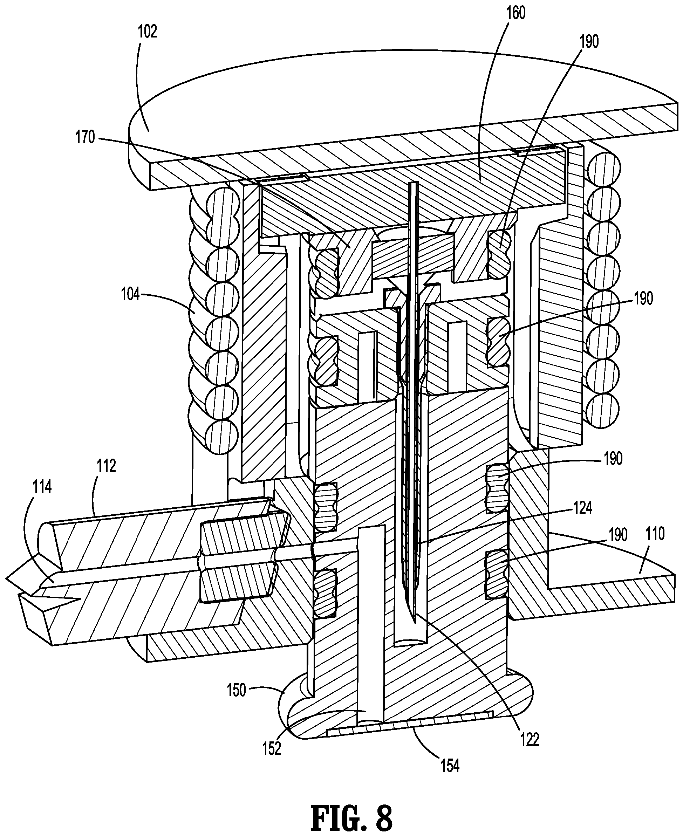

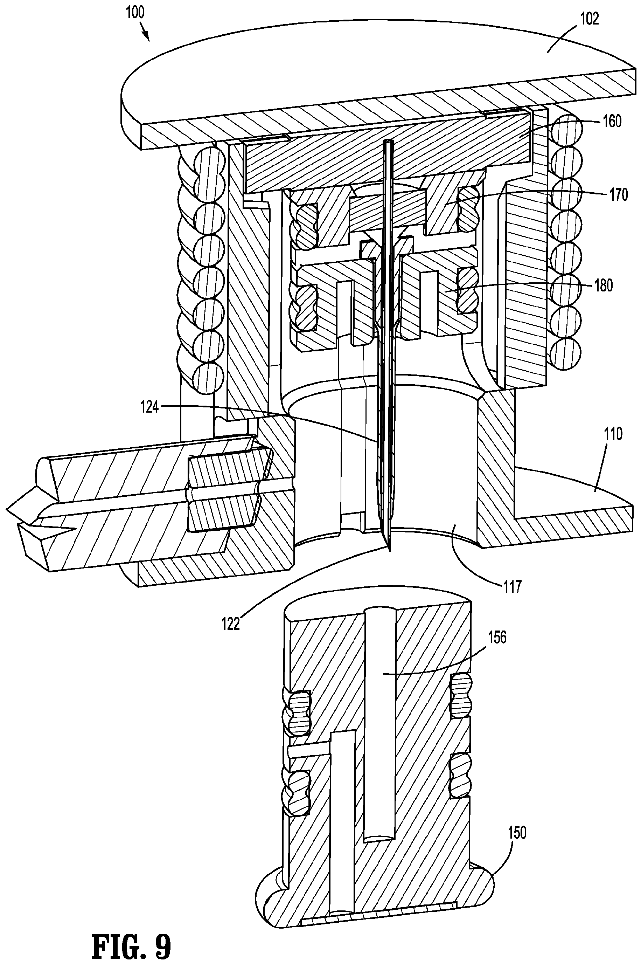

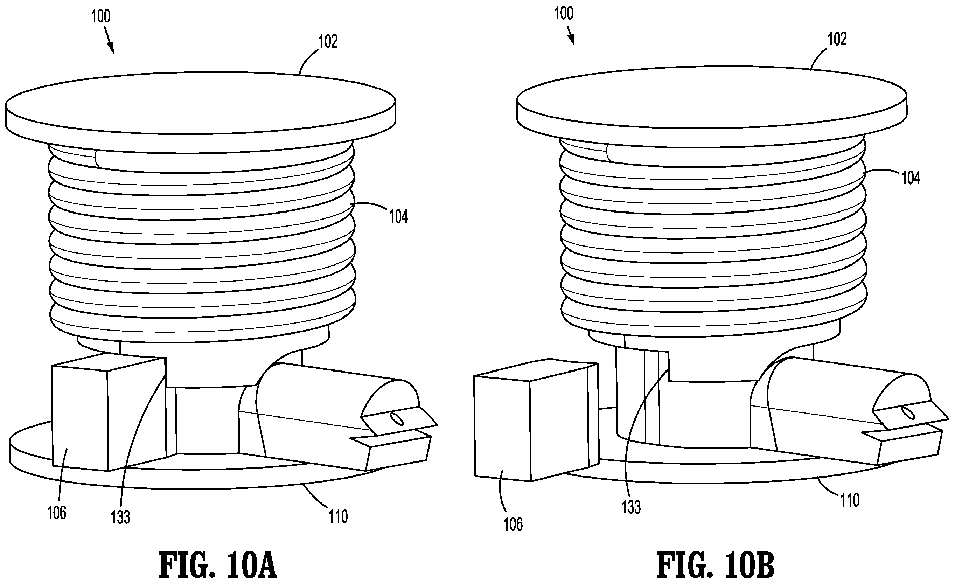

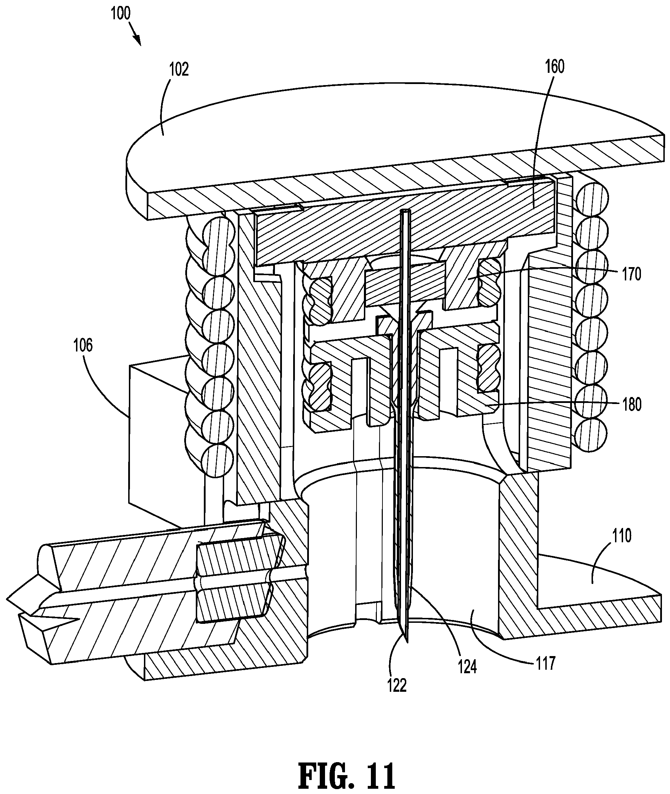

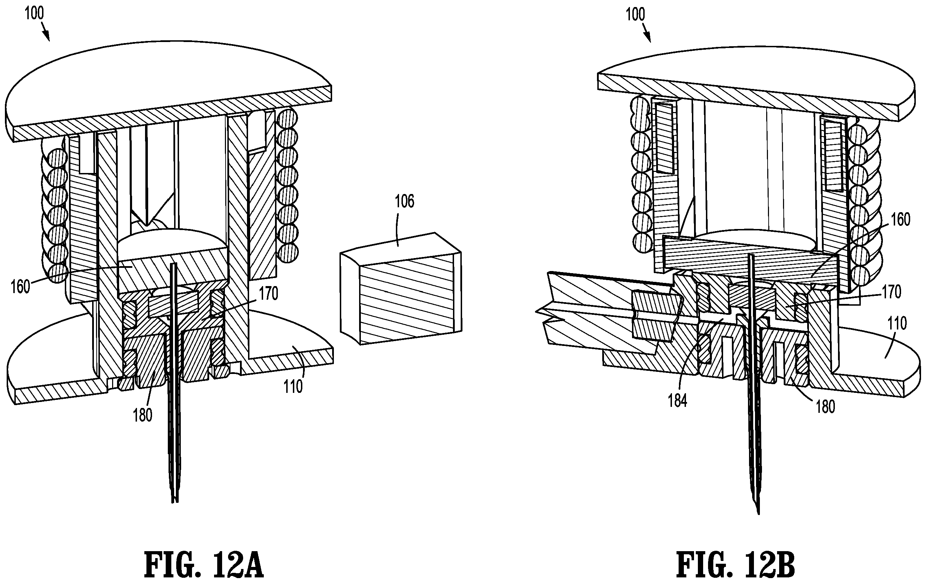

FIGS. 8-15 show progressive views of the operation of the torsional insertion mechanism 100 of FIG. 1. Initially, the torsional insertion mechanism 100 includes the PGUP 150 installed in the lower housing 110 (FIG.8). In operation or use, the PGUP 150 is removed from the lower housing 110 to expose the introducer needle 122 (FIG. 9). The stop member 106 is disengaged from the bushing 130 of the torsional insertion mechanism 100 (FIGS. 10A and 10B). For example, a motor 1730 of an infusion pump system 1700 (FIG. 17) may be used to remove the stop member 106. In another example, a push/pull button (not shown) may be used to disengage the stop member 106. Once the stop member 106 is removed, the bushing 130 is no longer constrained against rotation and begins to rotate in response to the stored potential energy in the torsional spring 104 being converted to kinetic energy. The pre-loaded (e.g., pre-tensioned and/or torsioned or wound) torsion spring 104 starts to impart torque and/or rotation on the bushing 130 (FIG. 11). Next, the bushing 130 rotates in response to torque forces being imparted thereto by the torsional spring 104 (FIGS. 12A and 12B), and moved from a first bushing position to a second bushing position.

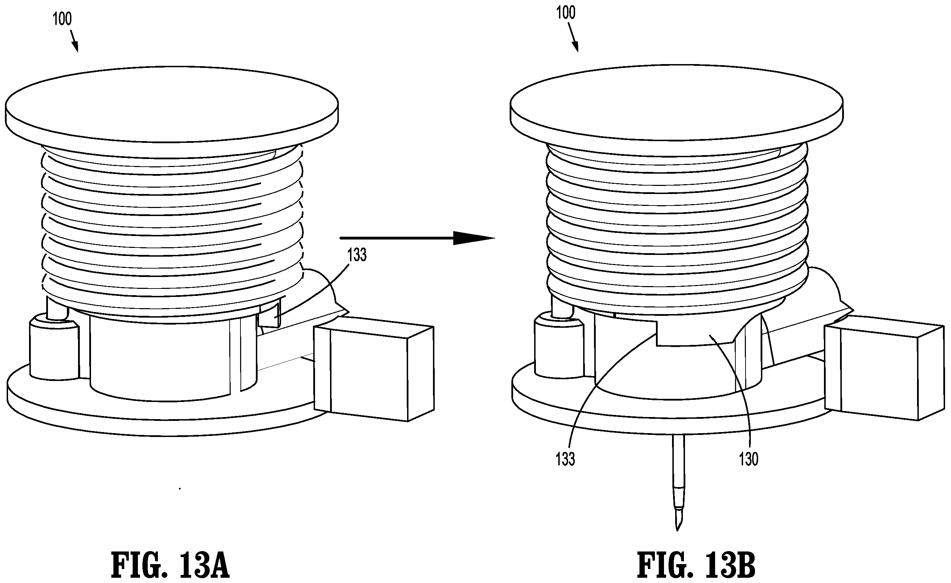

As the bushing 130 rotates, the bosses 162a, 162b of the insertion assembly 300 are moved in a vertical motion (e.g., downward in an insertion direction toward the skin of the patient) due to the interaction of the bosses 162a, 162b with/against the angled ramps 134a, 134b of the bushing 130. The angled ramps 134a, 134b push down the bosses 162a, 162b causing the insertion assembly 300 to move in the downward direction from a first insertion position (FIG. 13A) to a second insertion position (FIG. 13B), exposing the introducer needle 122 and cannula 124 from the bottom of the lower housing 110.

Referring to FIG. 14, a snap mechanism 200 is shown. In aspects, the snap mechanism 200 may hold the cannula carrier 180 and needle guide 170 in place when the insertion assembly 300 is in the second insertion position. (FIG. 14). The snap mechanism 200 may include fingers 202 that attach and/or snap into recesses 110b in the bottom of the lower housing 110. It is contemplated that any suitable method of attachment may be used.

Referring to FIG. 15, a fluid delivery path 184 of the torsional insertion mechanism 100 is shown. In the second insertion position, a fluid delivery path 184 is formed.

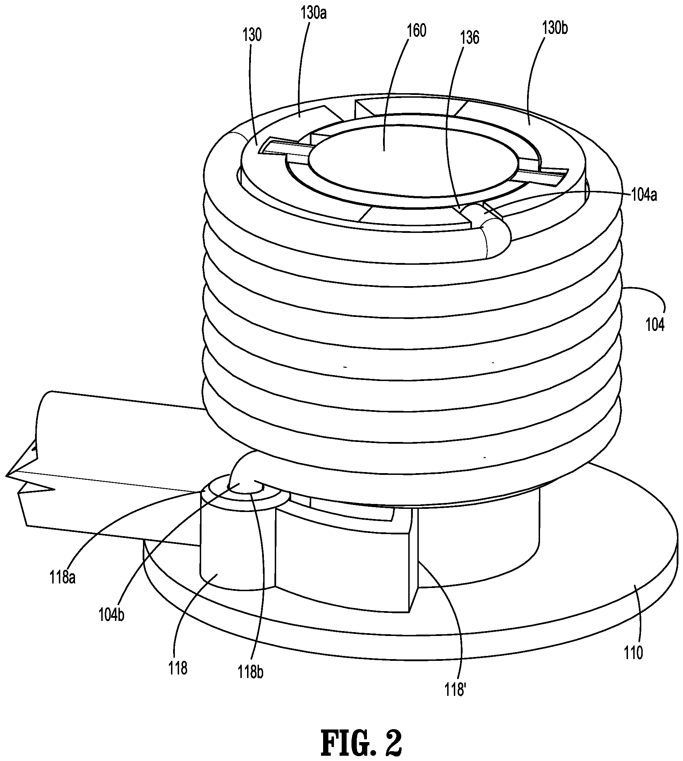

Referring to FIGS. 16A and 16B, as the bushing 130 rotates from a second bushing position (FIG. 16A) to a third bushing position (FIG. 16B) in response to the rotational motion imparted by the torsion spring 104, the introducer needle 122 retracts back into the insertion assembly 300. Since the introducer needle 122 is retracted, fluid delivery of the medicament (e.g., insulin) is enabled (FIG. 15). The rotational stop 118' (FIG. 2) stops further rotation of the bushing 120.

FIG. 17 shows an exemplary infusion pump system 1700, in accordance with aspects of the disclosure. The infusion pump system 1700 generally includes a torsional insertion mechanism 100 (FIG. 1), a medical reservoir 1720 in fluid communication with the insertion assembly 300 of the torsional insertion mechanism 100, and a motor 1730 configured to engage and/or disengage stop member 106.

The phrases "in an embodiment," "in embodiments," "in various embodiments," "in some embodiments," or "in other embodiments" may each refer to one or more of the same or different embodiments in accordance with the disclosure. A phrase in the form "A or B" means "(A), (B), or (A and B)." A phrase in the form "at least one of A, B, or C" means "(A); (B); (C); (A and B); (A and C); (B and C); or (A, B, and C)."

It should be understood that the foregoing description is only illustrative of the disclosure. To the extent consistent, any or all of the aspects detailed herein may be used in conjunction with any or all of the other aspects detailed herein. Various alternatives and modifications can be devised by those skilled in the art without departing from the disclosure. Accordingly, the disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

## Claims

1. A torsional insertion mechanism (100), comprising:
a torsion spring (104) configured to rotate a bushing (130) between a first spring position and a second spring position; and
an insertion assembly (300) configured to move from a first insertion position to a second insertion position in response to the rotation of the bushing (130).

2. The torsional insertion mechanism (100) according to claim 1, wherein the bushing (130) includes an inner surface (132a, 132b) including an angled ramp (134a, 134b).

3. The torsional insertion mechanism (100) according to claim 2, wherein the insertion assembly (300) includes a tubular boss (162a, 162b) configured to contact the angled ramp (134a, 134b) and move from a first boss position to a second boss position in response to the rotation of the bushing (130).

4. The torsional insertion mechanism (100) according to claim 2 or 3, wherein the angled ramp (134a, 134b) contacts the insertion assembly (300).

5. The torsional insertion mechanism (100) according to any of claims 2 to 4, wherein the insertion assembly (300) includes a captive introducer needle (122), and a cannula (124) configured for insertion in tissue in response to rotational motion of the bushing (130).

6. The torsional insertion mechanism (100) according to claim 5, wherein the angled ramp (134a, 134b) includes a track (132c) that pushes the captive introducer needle (122) down and/or pulls the captive introducer needle (122) out in response to rotation of the bushing (130).

7. The torsional insertion mechanism (100) according to any of claims 1 to 6, further comprising a stop member (106) configured to selectively prevent rotation of the bushing (130).

8. The torsional insertion mechanism (100) according to claim 7, wherein the stop member (106) is configured to move from a first stop position to prevent rotation of the bushing (130) by engaging a stop recess (133) in an outer surface of the bushing (130), to a second stop position to enable rotation of the bushing by disengaging the stop recess (133) of the bushing (130).

9. The torsional insertion mechanism (100) according to claim 5, wherein the insertion assembly (300) further includes:
a cannula carrier (180) configured to capture the cannula (124);
a needle guide (170) configured to guide the cannula (124) in the cannula carrier (180); and
a fluid flow path (184) that passes through the needle guide (170) to the cannula (124) in the cannula carrier (180), wherein the fluid flow path is configured for fluid communication between the cannula (124) and a medical reservoir (1720).

10. The torsional insertion mechanism (100) according to claim 9, wherein the needle guide (170) further includes a tubular boss (162a, 162b) extended from a bottom of the needle guide (170),
wherein the cannula carrier (180) includes a bore (182); and
wherein the cannula (124) is captured in a radial gap between the bore (182) in the cannula carrier (180) and the tubular boss (162a, 162b).

11. The torsional insertion mechanism (100) according to claim 5, wherein the introducer needle (122) and the cannula (124) are configured to move from a first needle position to a second needle position in response to the insertion assembly moving from the first insertion position to the second insertion position.

12. The torsional insertion mechanism (100) according to claim 11, wherein the introducer needle (122) is configured to move to the first needle position from the second needle position and the cannula remains in the second needle position in response to the insertion assembly (300) moving from the second insertion position to a third insertion position.

13. An infusion pump system (1700), comprising:
the torsional insertion mechanism (100) according to any of claims 2 to 6; the torsional insertion mechanism (100) further including a stop member (106)
configured to at least one of enable or disable rotation of the bushing (130);
a medical reservoir (1720) in fluid communication with the insertion assembly (300); and
a motor (1730) configured to at least one of engage or disengage the stop member (106).

14. A method for operating a torsional inserter (100) of an insulin infusion system (1700), the method comprising:
rotating a bushing (130) between a first spring position and a second spring position by a torsion spring (104); and
moving an insertion assembly (300) from a first insertion position to a second insertion position in response to the rotation of the bushing (130).

15. The method according to claim 14, further comprising:
moving an introducer needle (122) and a cannula (124) from a first needle position to a second needle position in response to the insertion assembly moving from the first insertion position to the second insertion position; and
optionally, moving the introducer needle (122) to the first needle position from the second needle position and the cannula (124) remaining in the second needle position in response to the insertion assembly (300) moving from the second insertion position to a third insertion position.
